# EUROPEAN PATENT SPECIFICATION

(11) **EP 4 010 053 B1**
(45) Date of publication and mention of the grant of the patent: **30.09.2026**
(21) Application number: 19753433.2
(22) Date of filing: 06.08.2019
(51) Int. Cl.: A61M 11/00, A24F 47/00, A61M 11/04, A61M 15/06

(54) **HYBRID AEROSOL PROVISION SYSTEMS**
HYBRIDE AEROSOLBEREITSTELLUNGSSYSTEME
SYSTÈME DE FOURNITURE D'AÉROSOL HYBRIDE

(43) Date of publication of application: 15.06.2022
(73) Proprietor: Nicoventures Trading Limited, London WC2R 3LA (GB)
(72) Inventor: POYNTON, Simon, London WC2R 3LA (GB); POTTER, Mark, London WC2R 3LA (GB); YILMAZ, Ugurhan, London WC2R 3LA (GB); CHEN, Shixiang, London WC2R 3LA (GB)
(74) Representative: D Young & Co LLP
(86) International application number: PCT/GB2019/052208
(87) International publication number: WO 2021/023953

(56) References cited:
- WO-A1-2014/150131
- WO-A1-2016/020675
- WO-A1-2016/062777
- WO-A1-2019/081571

## Description

### Field

The present disclosure relates to aerosol provision systems such as nicotine delivery systems (e.g. electronic cigarettes and the like).

### Background

Electronic aerosol provision systems such as electronic cigarettes (e-cigarettes) generally contain a vapour precursor material, such as a reservoir of a source liquid containing a formulation, typically including nicotine, or a solid material such as a tobacco-based product, from which a vapour is generated for inhalation by a user, for example through heat vaporisation. Thus, a aerosol provision system will typically comprise a vapour generation chamber containing a vaporiser, e.g. a heating element, arranged to vaporise a portion of precursor material to generate a vapour in the vapour generation chamber. As a user inhales on the device and electrical power is supplied to the vaporiser, air is drawn into the device through inlet holes and into the vapour generation chamber where the air mixes with the vaporised precursor material and forms a condensation aerosol. There is a flow path between the vapour generation chamber and an opening in the mouthpiece so the incoming air drawn through the vapour generation chamber continues along the flow path to the mouthpiece opening, carrying some of the vapour / condensation aerosol with it, and out through the mouthpiece opening for inhalation by the user.

Some electronic cigarettes may also include an aerosol modifying material in the flow path through the device to modify the aerosol, e.g., via imparting additional flavours to the aerosol. Such devices may sometimes be referred to as hybrid devices and the aerosol modifying element may, for example, include a portion of tobacco arranged in the air path between the vapour generation chamber and the mouthpiece so that vapour / condensation aerosol drawn through the devices passes through the portion of tobacco before exiting the mouthpiece for user inhalation.

In such hybrid systems, the effectiveness of the aerosol modifying material to impart additional flavours or the like to the aerosol is dependent in part on the temperature of the tobacco material. However, in some systems, the aerosol modifying material is provided as a "bolt-on" to an existing electronic cigarette, and the overall system is not optimised to deliver a more satisfactory aerosol to a user.

Various approaches are described herein which seek to provide improved performance of the device while helping address or mitigate some of the issues discussed above.

### Summary

Respective aspects and features of the present disclosure are defined in the appended claims.

### Brief Description of the Drawings

Embodiments of the invention will now be described, by way of example only, with reference to the accompanying drawings, in which:
Figure 1 represents in highly schematic cross-sectional view of a hybrid aerosol provision system in accordance with certain embodiments of the disclosure;
Figure 2 shows a highly schematic cross-sectional view of the cartridge part of the hybrid aerosol provision system of Figure 1;
Figure 3 shows a highly schematic cross-sectional view of a removable insert part which can be used with the hybrid aerosol provision system 1 of Figure 1; and
Figure 4 represents an exemplary method for generating an aerosol which has been modified using a hybrid aerosol provision system, such as the hybrid aerosol provision system of Figure 1.

### Detailed Description

Aspects and features of certain examples and embodiments are discussed / described herein. Some aspects and features of certain examples and embodiments may be implemented conventionally and these are not discussed / described in detail in the interests of brevity. It will thus be appreciated that aspects and features of apparatus and methods discussed herein which are not described in detail may be implemented in accordance with any conventional techniques for implementing such aspects and features.

The present disclosure relates to non-combustible aerosol provision systems, which may also be referred to as aerosol provision systems, such as e-cigarettes, including hybrid devices. Throughout the following description the term "e-cigarette" or "electronic cigarette" may sometimes be used, but it will be appreciated this term may be used interchangeably with aerosol provision system / device and electronic aerosol provision system / device. Furthermore, and as is common in the technical field, the terms "vapour" and "aerosol", and related terms such as "vaporise", "volatilise" and "aerosolise", may generally be used interchangeably.

According to the present disclosure, a "non-combustible" aerosol provision system is one where a constituent aerosolisable material of the aerosol provision system (or component thereof) is not combusted or burned in order to facilitate delivery to a user.

In some embodiments, the non-combustible aerosol provision system is a hybrid system to generate aerosol using a combination of aerosolisable materials, one or a plurality of which may be heated. Each of the aerosolisable materials may be, for example, in the form of a solid, liquid or gel and may or may not contain nicotine. In some embodiments, the hybrid system comprises a liquid or gel aerosolisable material and a solid aerosolisable material. The solid aerosolisable material may comprise, for example, tobacco or a non-tobacco product.

Typically, the non-combustible aerosol provision system may comprise a non-combustible aerosol provision device and an article for use with the non-combustible aerosol provision device. However, it is envisaged that articles which themselves comprise a means for powering an aerosol generating component may themselves form the non-combustible aerosol provision system.

In some embodiments, the non-combustible aerosol provision device may comprise a power source and a controller. The power source may, for example, be an electric power source or an exothermic power source. In some embodiments, the exothermic power source comprises a carbon substrate which may be energised so as to distribute power in the form of heat to an aerosolisable material or heat transfer material in proximity to the exothermic power source. In some embodiments, the power source, such as an exothermic power source, is provided in the article so as to form the non-combustible aerosol provision.

In some embodiments, the article for use with the non-combustible aerosol provision device may comprise an aerosolisable material, an aerosol generating component, an aerosol generating area, a mouthpiece, and/or an area for receiving aerosolisable material.

In some embodiments, the aerosol generating component is a heater capable of interacting with the aerosolisable material so as to release one or more volatiles from the aerosolisable material to form an aerosol.

In some embodiments, the substance to be delivered may be an aerosolisable material or a non-aerosolisable material. As appropriate, either material may comprise an active constituent, a carrier constituent and optionally one or more other functional constituents and/or one or more flavours.

The active constituent may comprise one or more physiologically and/or olfactory active constituents which are included in the aerosolisable material in order to achieve a physiological and/or olfactory response in the user. The active constituent may for example be selected from nutraceuticals, nootropics, and psychoactives. The active constituent may be naturally occurring or synthetically obtained. The active constituent may comprise for example nicotine, caffeine, taurine, theine, a vitamin such as B6 or B12 or C, melatonin, a cannabinoid, or a constituent, derivative, or combinations thereof. The active constituent may comprise a constituent, derivative or extract of tobacco or of another botanical such as cannabis, such as a cannabinoid or terpene. In some embodiments, the active constituent is a physiologically active constituent and may be selected from nicotine, nicotine salts (e.g. nicotine ditartrate/nicotine bitartrate), nicotine-free tobacco substitutes, other alkaloids such as caffeine, cannabinoids, or mixtures thereof.

In some embodiments, a "flavour" and/or "flavourant" (or sometimes flavour constituent) which, where local regulations permit, may be used to create a desired taste, aroma or other somatosensorial sensation in a product for adult consumers. In some instances such constituents may be referred to as flavours, flavourants, cooling agents, heating agents, or sweetening agents. They may include naturally occurring flavour materials, botanicals, extracts of botanicals, synthetically obtained materials, or combinations thereof (e.g., tobacco, cannabis, licorice (liquorice), hydrangea, eugenol, Japanese white bark magnolia leaf, chamomile, fenugreek, clove, maple, matcha, menthol, Japanese mint, aniseed (anise), cinnamon, turmeric, Indian spices, Asian spices, herb, wintergreen, cherry, berry, red berry, cranberry, peach, apple, orange, mango, clementine, lemon, lime, tropical fruit, papaya, rhubarb, grape, durian, dragon fruit, cucumber, blueberry, mulberry, citrus fruits, Drambuie, bourbon, scotch, whiskey, gin, tequila, rum, spearmint, peppermint, lavender, aloe vera, cardamom, celery, cascarilla, nutmeg, sandalwood, bergamot, geranium, khat, naswar, betel, shisha, pine, honey essence, rose oil, vanilla, lemon oil, orange oil, orange blossom, cherry blossom, cassia, caraway, cognac, jasmine, ylang-ylang, sage, fennel, wasabi, piment, ginger, coriander, coffee, hemp, a mint oil from any species of the genus Mentha, eucalyptus, star anise, cocoa, lemongrass, rooibos, flax, ginkgo biloba, hazel, hibiscus, laurel, mate, orange skin, rose, tea such as green tea or black tea, thyme, juniper, elderflower, basil, bay leaves, cumin, oregano, paprika, rosemary, saffron, lemon peel, mint, beefsteak plant, curcuma, cilantro, myrtle, cassis, valerian, pimento, mace, damien, marjoram, olive, lemon balm, lemon basil, chive, carvi, verbena, tarragon, limonene, thymol, camphene), flavour enhancers, bitterness receptor site blockers, sensorial receptor site activators or stimulators, sugars and/or sugar substitutes (e.g., sucralose, acesulfame potassium, aspartame, saccharine, cyclamates, lactose, sucrose, glucose, fructose, sorbitol, or mannitol), and other additives such as charcoal, chlorophyll, minerals, botanicals, or breath freshening agents. They may be imitation, synthetic or natural ingredients or blends thereof. They may be in any suitable form, for example, liquid such as an oil, solid such as a powder, or gasone or more of extracts (e.g., licorice, hydrangea, Japanese white bark magnolia leaf, chamomile, fenugreek, clove, menthol, Japanese mint, aniseed, cinnamon, herb, wintergreen, cherry, berry, peach, apple, Drambuie, bourbon, scotch, whiskey, spearmint, peppermint, lavender, cardamom, celery, cascarilla, nutmeg, sandalwood, bergamot, geranium, honey essence, rose oil, vanilla, lemon oil, orange oil, cassia, caraway, cognac, jasmine, ylang-ylang, sage, fennel, piment, ginger, anise, coriander, coffee, or a mint oil from any species of the genus Mentha), flavour enhancers, bitterness receptor site blockers, sensorial receptor site activators or stimulators, sugars and/or sugar substitutes (e.g., sucralose, acesulfame potassium, aspartame, saccharine, cyclamates, lactose, sucrose, glucose, fructose, sorbitol, or mannitol), and other additives such as charcoal, chlorophyll, minerals, botanicals, or breath freshening agents. They may be imitation, synthetic or natural ingredients or blends thereof. They may be in any suitable form, for example, oil, liquid, or powder.

In some embodiments, the flavour may comprise a sensate, which is intended to achieve a somatosensorial sensation which are usually chemically induced and perceived by the stimulation of the fifth cranial nerve (trigeminal nerve), in addition to or in place of aroma or taste nerves, and these may include agents providing heating, cooling, tingling, numbing effect. A suitable heat effect agent may be, but is not limited to, vanillyl ethyl ether and a suitable cooling agent may be, but not limited to eucalyptol, WS-3.

The carrier constituent may comprise one or more constituents capable of forming an aerosol. In some embodiments, the carrier constituent may comprise one or more of glycerine, glycerol, propylene glycol, diethylene glycol, triethylene glycol, tetraethylene glycol, 1,3-butylene glycol, erythritol, meso-Erythritol, ethyl vanillate, ethyl laurate, a diethyl suberate, triethyl citrate, triacetin, a diacetin mixture, benzyl benzoate, benzyl phenyl acetate, tributyrin, lauryl acetate, lauric acid, myristic acid, and propylene carbonate.

The one or more other functional constituents may comprise one or more of **pH** regulators, colouring agents, preservatives, binders, fillers, stabilizers, and/or antioxidants.

In some embodiments, the article for use with the non-combustible aerosol provision device may comprise aerosolisable material or an area for receiving aerosolisable material. In some embodiments, the article for use with the non-combustible aerosol provision device may comprise a mouthpiece. The area for receiving aerosolisable material may be a storage area for storing aerosolisable material. For example, the storage area may be a reservoir. In some embodiments, the area for receiving aerosolisable material may be separate from, or combined with, an aerosol generating area.

Aerosolisable material, which also may be referred to herein as aerosol generating material or aerosol precursor material, is material that is capable of generating aerosol, for example when heated, irradiated or energized in any other way. Aerosolisable material may, for example, be in the form of a solid, liquid or gel which may or may not contain nicotine and/or flavourants. In some embodiments, the aerosolisable material may comprise an "amorphous solid", which may alternatively be referred to as a "monolithic solid" (i.e. non-fibrous). In some embodiments, the amorphous solid may be a dried gel. The amorphous solid is a solid material that may retain some fluid, such as liquid, within it. In some embodiments, the aerosolisable material may for example comprise from about 50wt%, 60wt% or 70wt% of amorphous solid, to about 90wt%, 95wt% or 100wt% of amorphous solid. As used herein, aerosolisable material may refer to material which includes any one or a combination of an active constituent, a carrier constituent, and an other functional constituent and/or flavour. Embodiments of the present disclosure include aerosoliable material which comprises only one or more carrier constituents.

The active constituent is or may comprise a substance considered to be a physiologically and/or olfactory active constituent which is included in the aerosolisable material in order to achieve a physiological and/or olfactory response. The active constituent includes any of the active constituents listed above.

The carrier constituent may comprise one or more constituents capable of forming an aerosol. In some embodiments, the carrier constituent may comprise one or more of glycerine, glycerol, propylene glycol, diethylene glycol, triethylene glycol, tetraethylene glycol, 1,3-butylene glycol, erythritol, meso-Erythritol, ethyl vanillate, ethyl laurate, a diethyl suberate, triethyl citrate, triacetin, a diacetin mixture, benzyl benzoate, benzyl phenyl acetate, tributyrin, lauryl acetate, lauric acid, myristic acid, and propylene carbonate.

The one or more other functional constituents may comprise one or more of **pH** regulators, colouring agents, preservatives, binders, fillers, stabilizers, and/or antioxidants.

Aerosol provision systems (e-cigarettes) often, though not always, comprise a modular assembly including both a reusable part and a replaceable (disposable) cartridge part. Often the replaceable cartridge part will comprise the vapour precursor material and the vaporiser and the reusable part will comprise the power supply (e.g. rechargeable battery), activation mechanism (e.g. button or puff sensor), and control circuitry. However, it will be appreciated these different parts may also comprise further elements depending on functionality. For example, for a hybrid device the cartridge part may also comprise the additional flavour element or flavour imparting medium, e.g. a portion of tobacco. In such cases the flavour element insert may itself be removable from the disposable cartridge part so it can be replaced separately from the cartridge, for example to change flavour or because the usable lifetime of the flavour element insert is less than the usable lifetime of the vapour generating components of the cartridge. In some examples the flavour element insert may be contained within a pod, container or further cartridge. In some examples, the pod may be reusable and a user may be able to access flavour element insert within the pod to replace the flavour element insert. In other examples, the pod may be disposable and a user is discouraged from accessing or attempting to replace the flavour element insert. Use of a pod may provide an enhanced user experience by, for example, ensuring optimal positioning of the flavour element insert within an airflow path and/or by restricting the properties of the flavour element insert (e.g. volume, consistency, density etc.).

The reusable device part will often also comprise additional components, such as a user interface for receiving user input and displaying operating status characteristics.

For modular devices a cartridge and control unit are electrically and mechanically coupled together for use, for example using a screw thread, latching or bayonet fixing with appropriately engaging electrical contacts. When the vapour precursor material in a cartridge is exhausted, or the user wishes to switch to a different cartridge having a different vapour precursor material, a cartridge may be removed from the control unit and a replacement cartridge attached in its place. Devices conforming to this type of two-part modular configuration may generally be referred to as two-part devices or multi-part devices.

It is relatively common for electronic cigarettes, including multi-part devices, to have a generally elongate shape and, for the sake of providing a concrete example, certain embodiments of the disclosure described herein will be taken to comprise a generally elongate multi-part device employing disposable cartridges with a tobacco pod insert. However, it will be appreciated the underlying principles described herein may equally be adopted for different electronic cigarette configurations, for example single-part devices or modular devices comprising more than two parts, refillable devices and single-use disposable devices, as well as devices conforming to other overall shapes, for example based on so-called box-mod high performance devices that typically have a more box-like shape. More generally, it will be appreciated certain embodiments of the disclosure are based on electronic cigarettes that are configured to provide activation functionality in accordance with the principles described herein, and the specific constructional aspects of electronic cigarette configured to provide the described activation functionality are not of primary significance.

The present disclosure relates primarily to hybrid aerosol provision systems in which a aerosol precursor material (e.g., a liquid) which may include any one or more of a active constituent, a carrier constituent, or an other functional constituent, is directly heated to generate an aerosol which is subsequently passed through or over an aerosol modifying material, e.g., a tobacco material or tobacco containing material, which modifies the properties of the generated aerosol (e.g., it imparts a flavour and/or nicotine to the aerosol).

In accordance with the present disclosure, it has been found the tobacco material may be heated to a suitable degree using the generated aerosol to help improve sensory performance (and more specifically to improve the flavour and/or nicotine uptake in the generated aerosol). More specifically, the lower surface of an insert containing or comprising the aerosol modifying material can be heated to between 50 °C to 150 °C, or between 70 °C to 140 °C, or between 85 °C to 125 °C, or between 100 °C to 125 °C, to provide an improved sensory performance to the user, particularly when heating an insert comprising tobacco. The temperature measurement may be made by obtaining the maximum temperature at the lower surface of the insert (that is, the surface of the insert closest to the heater) over 50 puffs made in accordance with the Coresta Recommended Method Number 81. In other implementations, this could be an average of the maximum temperatures for each of the 50 puffs. The heating is controlled by appropriately setting the distance from the heater to the lower surface of the insert and/or by heating a pre-determined mass to deliver energy to the lower surface of the insert. By adjusting one or both of these parameters, the user may experience a good sensory performance while not compromising the lifetime of the device (e.g., in respect of battery life). It should also be appreciated that the type of aerosol precursor material which is to be vaporised may also affect the temperature of the lower surface of the insert, as different compositions may have different abilities to transport different amounts of energy.

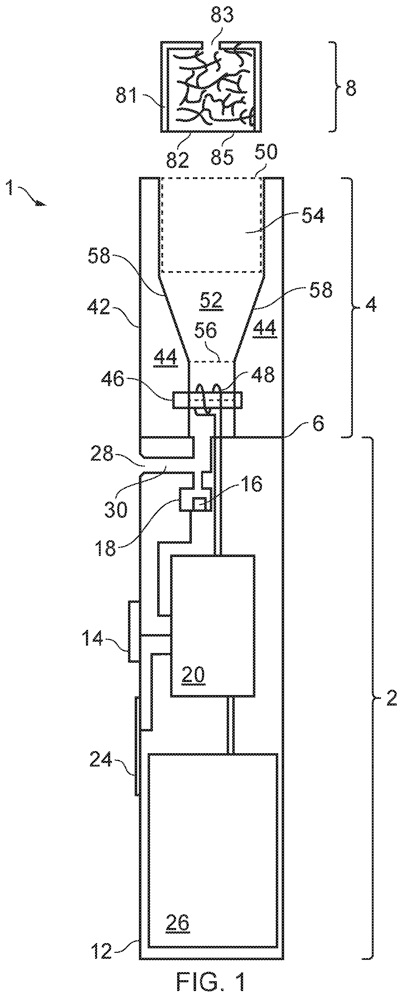

Figure 1 is a cross-sectional view through an example hybrid aerosol provision device 1 in accordance with certain embodiments of the disclosure. The hybrid aerosol provision device 1 comprises two main components, namely a reusable part 2 and a replaceable / disposable cartridge part 4 (sometimes referred to herein as an aerosol precursor material part or an aerosol precursor material storing part). In this specific example, the cartridge part 4 includes a removable insert 8 (sometimes referred to herein as an aerosol modifying material part or aerosol modifying material storage part). In normal use the reusable part 2 and the cartridge part 4 are releasably coupled together at an interface 6. When the cartridge part is exhausted or the user simply wishes to switch to a different cartridge part, the cartridge part may be removed from the reusable part and a replacement cartridge part attached to the reusable part in its place. The interface 6 provides a structural, electrical and air path connection between the two parts and may be established in accordance with conventional techniques, for example based around a screw thread, latch mechanism, or bayonet fixing with appropriately arranged electrical contacts and openings for establishing the electrical connection and air path between the two parts as appropriate. The specific manner by which the cartridge part 4 mechanically mounts to the reusable part 2 is not significant to the principles described herein, but for the sake of a concrete example is assumed here to comprise a latching mechanism, for example with a portion of the cartridge being received in a corresponding receptacle in the reusable part with cooperating latch engaging elements (not represented in Figure 1). It will also be appreciated the interface 6 in some implementations may not support an electrical connection between the respective parts. For example, in some implementations a vaporiser may be provided by the reusable part rather than in the cartridge part, or the transfer of electrical power from the reusable part to the cartridge part may be wireless (e.g. based on electromagnetic induction), so that an electrical connection between the reusable part and the cartridge part is not needed. In these implementations, the element within the cartridge part that heats up may be referred to as a susceptor.

In Figure 1, the cartridge part 4 comprises a cartridge housing 42 formed of a plastics material. The cartridge housing 42 supports other components of the cartridge part and provides the mechanical interface 6 with the reusable part 2. The cartridge housing is generally circularly symmetric about a longitudinal axis along which the cartridge part couples to the reusable part 2. In this example the cartridge part has a length of around 4 cm and a diameter of around 3 cm. However, it will be appreciated the specific geometry, and more generally the overall shapes and materials used, may be different in different implementations.

Within the cartridge housing 42 is a reservoir 44 that contains liquid aerosol precursor material. The liquid aerosol precursor material may be conventional, and may be referred to as e-liquid. The liquid reservoir 44 in this example has an annular shape with an outer wall defined by the cartridge housing 42 and an inner wall 58 that defines an air path 52 through the cartridge part 4. The reservoir 44 is closed at each end with end walls to contain the e-liquid. The reservoir 44 may be formed in accordance with conventional techniques, for example it may comprise a plastics material and be integrally moulded with the cartridge housing 42. It should be appreciated that in other implementations, the cartridge part 4 may be configured to store any suitable aerosol precursor material, e.g., a solid or gel, as desired.

The removable insert 8 in this example is inserted into an open end of air path 52 opposite to the end of the cartridge 4 which couples to the control unit 2. The region of the cartridge air path 52 into which the removable insert 8 is inserted in effect defines an insert region 54 for the cartridge part. In these and other examples, the retention and positioning of the removable insert 8 may be due to friction and/or may be facilitated by clips, ledges and other features within the air path 52. In some examples the removable insert 8 may be further retained by attaching a mouthpiece element downstream of the removable insert 8. Such a mouthpiece element would include an opening at each end to allow air drawn along the air path 52 during use.

In the example shown, the removable insert 8 includes a housing which houses or retains an aerosol modifying material. For example, the aerosol modifying material may be any suitable material which modifies a property of an aerosol that passes over/through the aerosol modifying material, such as a botanical, flavour, active content (e.g., nicotine content), acid, base or physical parameters of the aerosol such as particle size. In the present disclosure, the aerosol modifying material is a portion of tobacco (for example shredded, reconstituted or extruded tobacco) which modifies an aerosol passing through the portion of tobacco by imparting flavour and nicotine to the aerosol. The removable insert in this example may therefore be referred to as a tobacco insert or tobacco pod. The housing for the removable insert 8 also includes an opening at each end to allow air drawn along the air path 52 during use to pass through the insert 8 and so pick up flavours from the flavourant within (tobacco in this example) before exiting the cartridge part 4 through a mouthpiece outlet 50 for user inhalation. In some examples, the housing of the insert 8 may define or otherwise incorporate a mouthpiece element. In other examples the insert may not include housing. For example, in some implementations, the insert may be a filter material which incorporates a flavourant and/or active ingredient. In other examples, the insert 8 may comprise a material (e.g., flavourant), which may or may not be wrapped or coated in an aerosol permeable wrap or layer.

The cartridge part further comprises a wick 46 and a heater (vaporiser) 48 located towards an end of the reservoir 44 opposite to the mouthpiece outlet 50. In this example the wick 46 extends transversely across the cartridge air path 52 with its ends extending into the reservoir 44 of e-liquid through openings in the inner wall of the reservoir 44. The openings in the inner wall of the reservoir 44 are sized to broadly match the dimensions of the wick 46 to provide a reasonable seal against leakage from the liquid reservoir into the cartridge air path without unduly compressing the wick, which may be detrimental to its fluid transfer performance.

The wick 46 and heater 48 are arranged in the cartridge air path 52 such that a region of the cartridge air path 52 around the wick 46 and heater 48 in effect defines a vapour generating region or vaporisation region 56 for the cartridge part. The e-liquid in the reservoir 44 infiltrates the wick 46 through the ends of the wick extending into the reservoir 44 and is drawn along the wick by surface tension / capillary action (i.e. wicking). The heater 48 in this example comprises an electrically resistive wire coiled around the wick 46. In this example the heater 48 comprises a nickel chrome alloy (Cr20Ni80) wire or a nickel iron alloy wire and the wick 46 comprises a glass fibre bundle or cotton fibre bundle, but it will be appreciated the specific vaporiser configuration is not significant to the principles described herein. In use electrical power may be supplied to the heater 48 to vaporise an amount of e-liquid (vapour precursor material) drawn to the vicinity of the heater 48 by the wick 46. Vaporised e-liquid may then become entrained in air drawn along the cartridge air path from the vaporisation region through the insert 8 and out the mouthpiece outlet 50 for user inhalation.

The rate at which e-liquid is vaporised by the vaporiser (heater) 48 will depend on the amount (level) of power supplied to the heater 48 during use. Thus electrical power can be applied to the heater to selectively generate vapour from the e-liquid in the cartridge part 4, and furthermore, the rate of vapour generation can be changed by changing the amount of power supplied to the heater 48, for example through pulse width and/or frequency modulation techniques or by using a DC/DC converter or other similar component to provide a stable (constant) power to the heater.

The reusable part 2 comprises an outer housing 12 with an opening that defines an air inlet 28 for the e-cigarette, a battery 26 for providing operating power for the electronic cigarette, control circuitry 20 for controlling and monitoring the operation of the electronic cigarette, a user input button 14, an inhalation sensor (puff detector) 16, which in this example comprises a pressure sensor located in a pressure sensor chamber 18, and a visual display 24.

The outer housing 12 may be formed, for example, from a plastics or metallic material and in this example has a circular cross-sectional area generally conforming to the shape and size of the cartridge part 4 so as to provide a smooth transition between the two parts at the interface 6. In this example, the reusable part has a length of around 6 cm so the overall length of the e-cigarette when the cartridge part and reusable part are coupled together is around 10 cm. However and as already noted, it will be appreciated that the overall shape and scale of an electronic cigarette implementing an embodiment of the disclosure is not significant to the principles described herein.

The air inlet 28 connects to an air path 30 through the reusable part 2. The reusable part air path 30 in turn connects to the cartridge air path 52 across the interface 6 when the reusable part 2 and cartridge part 4 are connected together. The pressure sensor chamber 18 containing the pressure sensor 16 is in fluid communication with the air path 30 in the reusable part 2 (i.e. the pressure sensor chamber 18 branches off from the air path 30 in the reusable part 2). Thus, when a user inhales on the mouthpiece opening 50, there is a drop in pressure in the pressure sensor chamber 18 that may be detected by the pressure sensor 16 and also air is drawn in through the air inlet 28, along the reusable part air path 30, across the interface 6, through the aerosol generation region in the vicinity of the vaporiser 48 (where vaporised e-liquid becomes entrained in the air flow when the vaporiser is active), along the cartridge air path 52, and out through the mouthpiece opening 50 for user inhalation.

The battery 26 in this example is rechargeable and may be of a conventional type, for example of the kind normally used in electronic cigarettes and other applications requiring provision of relatively high currents over relatively short periods. The battery 26 may be recharged through a charging connector in the reusable part housing 12, for example a USB connector.

The user input button 14 in this example is a conventional mechanical button, for example comprising a spring mounted component which may be pressed by a user to establish an electrical contact. In this regard, the input button may be considered to provide a manual input mechanism for the terminal device, but the specific manner in which the button is implemented is not significant. For example, different forms of mechanical button or touch-sensitive button (e.g. based on capacitive or optical sensing techniques) may be used in other implementations. The specific manner in which the button is implemented may, for example, be selected having regard to a desired aesthetic appearance.

The display 24 is provided to give a user with a visual indication of various characteristics associated with the electronic cigarette, for example current power setting information, remaining battery power, and so forth. The display may be implemented in various ways. In this example the display 24 comprises a conventional pixilated LCD screen that may be driven to display the desired information in accordance with conventional techniques. In other implementations the display may comprise one or more discrete indicators, for example LEDs, that are arranged to display the desired information, for example through particular colours and / or flash sequences. More generally, the manner in which the display is provided and information is displayed to a user using the display is not significant to the principles described herein. Some embodiments may not include a visual display and may include other means for providing a user with information relating to operating characteristics of the electronic cigarette, for example using audio signalling or haptic feedback, or may not include any means for providing a user with information relating to operating characteristics of the electronic cigarette.

The control circuitry 20 is suitably configured / programmed to control the operation of the electronic cigarette to provide functionality in accordance with embodiments of the disclosure as described further herein, as well as for providing conventional operating functions of the electronic cigarette in line with the established techniques for controlling such devices. The control circuitry (processor circuitry) 20 may be considered to logically comprise various subunits / circuitry elements associated with different aspects of the electronic cigarette's operation in accordance with the principles described herein and other conventional operating aspects of electronic cigarettes, such as display driving circuitry and user input detection. It will be appreciated the functionality of the control circuitry 20 can be provided in various different ways, for example using one or more suitably programmed programmable computer(s) and / or one or more suitably configured application-specific integrated circuit(s) / circuitry / chip(s) / chipset(s) configured to provide the desired functionality.

In this example the hybrid aerosol provision system 1 comprises a user input button 14 and an inhalation sensor 16. The control circuitry 20 may be configured to receive signalling from the inhalation sensor 16 and to use this signalling to determine if a user is inhaling on the hybrid aerosol provision system 1 and also to receive signalling from the input button 14 and to use this signalling to determine if a user is pressing (i.e. activating) the input button. These aspects of the operation of the aerosol provision system 1 (i.e. puff detection and button press detection) may in themselves be performed in accordance with established techniques (for example using conventional inhalation sensor and inhalation sensor signal processing techniques and using conventional input button and input button signal processing techniques). Other example aerosol provision systems may have only one of a user input button 14 and an inhalation sensor 16. In further examples, a aerosol provision system may have neither a user input button or an inhalation sensor depending on the configuration and operation of the system.

The cross-sectional area of the air path 52 at a location can be defined as the area of the plane perpendicular or transverse to a central or medial axis of the air path at that location. The area may be bound by at least one wall, for example, or other structural features. In use, the air flows in the direction of the central axis from the air inlet 28 towards the air outlet 50. Hence, the cross-sectional area provides a measure of the transverse area available for air to flow through during use.

As shown in Figure 1, in this example, the air path 52 may have a change between the vapour generation region 56 and the insert region 54. Figure 1 shows an air path 52 that expands in cross-section towards the mouthpiece end 50. In other examples, the cross-section of the air path may be fixed or the cross-section of the air path may narrow. In examples, the wall (or walls) 58 defining the air path 52 may be shaped to provide a desired change in cross-sectional area. For example, the air path 52 between the vapour generation region 56 and the insert region 54 may be defined by a wall 58 which is a single continuous or near continuous wall (e.g. a cylindrical wall or similar).

The channel formed by the wall 58 between the vapour generation region 56 and the insert region 54 may be described as a funnel, expanding tube or hollow frustum; for example it may be described as having a frusto-conical or frusto-pyramidal shape. For example, this may be achieved by increasing or expanding the separation between opposing portions of the wall 58 relative to the distance downstream (conversely, decreasing or contracting the separation between opposing portions relative to the distance upstream). In relation to Figure 1, it will be appreciated that while the expansion in cross-sectional area appears to be in one dimension (i.e. across the page, as drawn), the expansion in cross-sectional area may be in both dimensions defining the plane perpendicular to the central or medial axis of the air path 52 (i.e. into the page as well as width across the page, as drawn).

In some examples the insert 8 may be provided in the form of a cartridge, container or pod comprising a housing 81 for retaining an aerosol modifying material (for example, a botanical component, in one example loose tobacco or tobacco granules). In some implementations, the tobacco granules may be alkaline treated granules to alter the pH of the tobacco and or the tobacco may be cut or ground tobacco. The housing may be formed from a plastics material, such as polypropylene, although in other implementations the housing 81 may be formed from other plastics materials, metals, or any other suitable material. The insert 8 further comprises an inlet 82 and an outlet 83 configured to allow aerosol to pass through the material contained within the insert 8. For example, in use the insert 8 is inserted or attached to the cartridge part 4 such that an aerosol produced by the heater 48 is drawn through the insert 8 in response to a user inhalation.

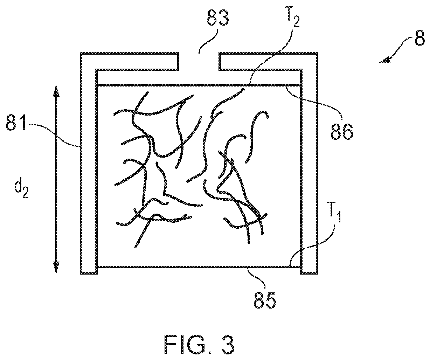

Where the insert 8 comprises a housing 81, the inlet 82 and/or the outlet 83 may be covered or otherwise comprise a mesh. For example, inlet 82 may comprise an inlet mesh 85. Meshes of these examples may allow vapour to infiltrate the insert 8 but retain the aerosol modifying material (for example, loose tobacco or tobacco granules) within the insert 8. By mesh it is meant a surface provided with a plurality of openings or holes. As examples, meshes may be provided by wire meshes, moulded meshes, machined meshes, or perforated surfaces. The mesh may be formed of a metal material, such as stainless steel, for example. Example meshes may have mesh holes of around 0.4mm with preferably separating spaces of 0.2mm between each hole. It will be appreciated that the size of the mesh holes may be dependent on the size of the aerosol modifying material. An outlet mesh may be constructed substantially similarly to an inlet mesh 85. In other examples, an outlet mesh may be configured differently to an inlet mesh 85. For example, the outlet mesh may be positioned at a distance away from the outlet 83 (see Figure 3, described later, for example).

In accordance with aspects of the present disclosure, the effectiveness by which the aerosol modifying material modifies the property or properties of an aerosol that passes through the aerosol modifying material may depends on a number of factors, and one of these factors may be the temperature of the aerosol modifying material. For example, the extent to which tobacco imparts flavour and/or nicotine to an aerosol passing through the tobacco is dependent in part on the temperature of the tobacco. Generally, a tobacco portion at a higher temperature will impart more flavour and/or more nicotine to an aerosol passing through the tobacco.

However, in the aforementioned example hybrid aerosol provision system shown in Figure 1, heating of tobacco within the insert 8 is performed indirectly. That is, the insert 8 does not include a dedicated heater for heating the tobacco. As a result, any heating of the tobacco is performed indirectly by the generated aerosol and/or heater 48 used to generate the aerosol.

As described, the cartridge part 4 includes a wick 46 and a heater 48 which is provided with electrical power to vaporise e-liquid held within the wick 46. The rate of vaporisation, that is the amount of vapour produced per second, is largely dependent on the power that is supplied to the heater 48. Assuming there is a relatively constant flow of e-liquid (that is, the wick is able to replenish any vaporised liquid at roughly the rate of vaporisation or greater), the temperature of the coil remains relatively constant during normal use. This is due in part to a cooling effect provided by the e-liquid as it replenishes vaporised e-liquid and due to the fact that energy is required to transform the state of the e-liquid (i.e., to transition to the vapour phase). For example, the temperature of the heater 48 during normal use may be in the range of 180 °C to 260 °C, but this will be dependent on the specific composition of the material (e-liquid) that is being vaporised. Altering the power to the heater 48 alters the vaporisation rate which correlates with the amount of aerosol produced. For a given puff, increasing the power generally increases the amount of aerosol produced for that puff. However, assuming the above conditions are maintained, (that is, the wick is able to replenish any vaporised liquid at roughly the rate of vaporisation or greater), the maximum temperature of the heater 48 remains fairly constant during normal use.

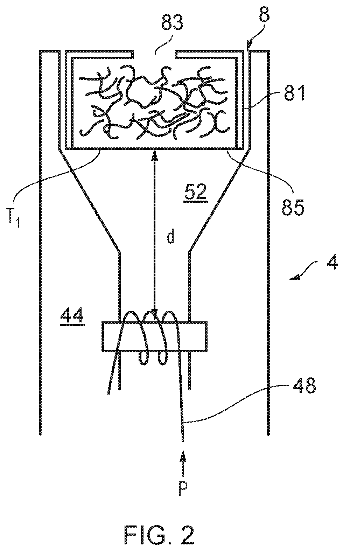

It has been found that at least two factors influence the temperature to which the tobacco insert 8 is heated during use of the hybrid aerosol provision system 1: the distance between the heater 48 and the tobacco insert 8, and the amount of aerosol generated per puff. Figure 2 is a schematic representation of a part of the cartridge part 4 of Figure 1, also shown in cross-section. Figure 2 further includes a double-headed arrow labelled d which signifies the distance between the heater 48 and the lower surface (i.e., the inlet mesh 85) of the tobacco insert 8. Figure 2 also includes an arrow labelled P signifying the power provided to the heater 48, e.g., via the control circuitry 20. As discussed below, the composition of the material to be vaporised may also be a factor in the temperature to which the tobacco insert is heated during use. In other implementations, the volume of the air path 52 and/or shape of the air path may also influence the temperature of the inlet mesh 85.

The inlet mesh 85 of the tobacco insert 8 is the first surface of the tobacco insert 8 present in the air path 52 and is therefore the part of the tobacco insert 8 that is closest to the heater 48. The inlet mesh 85 is downstream of the heater 48 along the air path 52 and therefore the relatively hot aerosol passes to / through the mesh 85. This causes the inlet mesh 85 to increase its temperature to a value T1 (as labelled in Figure 2). It should be appreciated that the temperature T1 of the inlet mesh 85 is a way of inferring the (average) temperature of tobacco material within the tobacco insert 8. The temperature within the tobacco insert 8 may not be the same as the temperature T1 of the insert mesh 8 as there may be different thermal energy transfers between the aerosol and the tobacco material, and the aerosol and the mesh 85, and it is thought that the temperature of the mesh 85 may be slightly higher than the maximum temperature of the tobacco material. It is also likely that the tobacco material may have a temperature gradient between the inlet and outlet of the tobacco insert 8, and thus the average temperature of the bulk tobacco material may be lower than the temperature T1 of the inlet mesh 85 of the insert 8. However, for the purposes of defining a repeatable and reliable temperature measurement, the temperature of the inlet mesh 85 is used herein.

It is theorised that the temperature T1 of the inlet mesh 85 (and thus the temperature of the tobacco material) is dependent in part on the properties of the aerosol itself when it passes through the inlet mesh 85. However, this may not be the only factor which influences or contributes to the temperature T1 of the inlet mesh 85. In some cases, energy from the heater 48 may be radiatively transferred directly from the heater 48 (i.e., via the mechanism of radiation). However, as discussed above, for any given system this contribution is approximately constant in normal use and is not considered to be the major factor that governs the temperature T1.

As mentioned above, when aerosol is generated at the heater 48, the temperature of the aerosol / vapour immediately after generation is approximately constant (when the rate of vaporisation is not greater than the rate of replenishment) and is governed predominantly by the composition of the e-liquid itself (i.e., the vaporisation temperature of the e-liquid). As explained, increasing the power to the heater 48 under the above conditions does not alter the temperature of the aerosol that is produced. Once the aerosol is generated, it travels along the air path 52 to the inlet mesh 85, and cools to form a condensation aerosol. Hence, in order to control the temperature T1 of the inlet mesh 85 during normal use, for a fixed distance d, it has been found that increasing the amount of aerosol produced (i.e., increasing the power P to the heater 48) leads to greater temperatures T1 at the inlet mesh 85. It is thought that this because an increased amount of aerosol generated imparts a greater amount of energy to the inlet mesh 85 as it passes through the inlet mesh 85, causing the temperature of the inlet mesh 85 to be comparatively greater. This can be thought of as the aerosol itself (i.e., the particles/droplets within) being the mechanism for transporting energy to the inlet mesh 85.

In this regard, it is thought that each unit mass of aerosol generated has a given energy associated therewith and, accordingly, increasing or decreasing the amount of aerosol produced increases or decreases the total amount of energy that is (or can be) transferred between the heater 48 and the lower mesh 85 of the tobacco insert 8. This energy is representative of the energy that is imparted to the tobacco material within the tobacco insert and thus the (average) temperature of tobacco material itself.

While each unit mass of aerosol may be produced with a certain (relatively constant) amount of energy, it should be appreciated that during transport of the aerosol from the location of generation (i.e., the vapour generation region 56) to the tobacco insert 8, energy is lost both to the surrounding cooler environment and during the condensation phase of the aerosol.

When considering the bulk aerosol per puff, energy may be lost at a certain rate which can be expressed as an energy loss per unit distance when considering aerosol moving at a fixed speed. Hence, broadly speaking, the larger the distance d between the heater 48 and the inlet mesh 85, the greater the energy loss, and hence a reduced total amount of energy which can be transferred to the tobacco material / inlet mesh 85.

It has been found that providing a temperature T1 of between 50 °C to 150 °C, or between 70 °C to 140 °C, or between 85 °C to 125 °C, or between 100 °C to 125 °C, leads to an improved user experience. In this regard, satisfactory user experience, while subjective, can generally be quantified by the amount of flavour and/or active ingredient (e.g., nicotine) contained in the aerosol after passing through the removable insert 8. The exact temperature T1 may depend on the type of material contained in the insert 8. For an implementation in which a tobacco material is contained within the removable insert 8, it has been found that a temperature T1 of between 85 °C to 125 °C, or between 100 °C to 125 °C, leads to a satisfactory user experience (i.e., perceived effect) when using the hybrid aerosol provision system 1. Generally, an increased level of flavour and/or nicotine leads to an improved user experience, although this will vary from user to user. Compared to systems in which tobacco is heated to a relatively low temperature, e.g., 30 °C, a temperature T1 of between 85 °C to 125 °C provides a greater amount of flavour and/or nicotine in the aerosol which is inhaled by the user.

Accordingly, the amount of aerosol generated and/or the distance d between the heater 48 and the insert 8 can be set for a given system such that a temperature T1 of between 50 °C to 150 °C of the lower surface of the insert 8 can be achieved, which results in an improved user experience. Providing a temperature T1 below 50 °C leads to a reduced perceived sensory experience as the amount of flavour and/or nicotine released from the tobacco is relatively low. Providing a temperature above 150 °C may lead to inefficiencies for the hybrid aerosol provision system 1 and/or a degradation in the perceived sensory effect, as explained in more detail below. Thus, the temperature range for T1 of between 50 °C to 150 °C, or between 70 °C to 140 °C, or more specifically between 85 °C to 125 °C, leads to a good overall sensory experience while maintaining an efficient system 1.

It should be appreciated that while, generally speaking, heating tobacco to higher temperatures is thought to lead to more flavour and/or nicotine being released from the tobacco, the mechanism by which the mesh 85 in the hybrid aerosol provision system 1 is heated is relatively inefficient, as compared to directly heating the tobacco portion for example. For instance, in systems which directly heat the tobacco, a dedicated heater is provided. However, in the hybrid aerosol provision systems 1 described, the heating of tobacco is essentially a by-product of the aerosol generated from the e-liquid. Therefore, it should be appreciated that while one could, in theory, increase the amount of aerosol generated by increasing the power supplied to the heater, the expected sensory gain may outweigh the required energy cost to do so, which ultimately impacts the number of uses (inhalations) of the hybrid aerosol provision device for a given battery size. In other words, the power P is not thought to map linearly to the expected temperature T1 of the mesh 85. Hence, increasing the power by e.g., 10%, one would expect a comparatively lower increase in the temperature T1 (and/or a comparative increase in the amount of flavour and/or nicotine entrained in the aerosol exiting the insert 8). Thus, a balance is struck, from an energy efficiency point of view. Although it will largely be dependent on the specifics of the hybrid aerosol provision system 1 in question, the power P to be delivered to a vaporiser which heats a material to be vaporised (e.g., a wick and coil heating an e-liquid as described above) will be on the order of 7.5 Watts, for example between 6.0 W to 9.0 W to provide a compromise between battery lifetime and sensory performance.

Additionally, increasing the power P to the heater leads to an increase in the mass of aerosol produced, as explained above. However, a physical limit on the mass of aerosol a user may want to inhale may also be taken into consideration. If the mass of aerosol is too high, some user's may experience unpleasant sensory effects. In one implementation, the mass of aerosol is set to be no greater than 12 mg per puff. Conversely, it should be appreciated that in order to achieve a certain temperature T1, as described above, a minimum amount of aerosol may be produced for a given system, which may be greater than 3 mg per puff, e.g., 4 mg per puff. Setting a mass of the aerosol generated (and hence power P to the heater) of between 4mg to 12 mg per puff has been found to be particularly suitable for a removable insert 8 comprising tobacco. A puff for the purposes of this disclosure is defined based on a standard puff or puffing regime which may be implemented via suitable puffing machine, for example, a Brogwaldt four-port smoke machine, manufactured by Brogwaldt. More particularly, a puff is defined as a 55 ml flow rate, over a 3 second period. This is consistent with the smoking method Coresta Recommended Method Number 81 (CRM 81).

Also, the maximum power P that can be applied may also be dictated by the rate of replenishment of the e-liquid in the wick 46. As is known, when the wick becomes dry (that is, the majority of the e-liquid in the wick is vaporised), the cooling effect of the e-liquid is reduced which can lead to an increase in the temperature of the heater 48. This can further result in undesirable effects, such as charring of the wick 46, which may cause damage to the cartridge part 4 and/or provide undesirable tastes that are perceived by the user.

Hence, these factors may be taken into account when setting the amount of aerosol to be generated. However, as noted above, this is only one factor and the distance d between the heater 48 and insert 8 should also be taken into account. While the above description is suggestive of minimising the distance d between the heater 48 and tobacco insert 8 to lead to greater temperatures of the tobacco insert 8, depending upon the materials used for the housing 81, for example, polypropylene, the distance should be no greater than 3 mm, and preferably between 3 to 10 mm, so as to avoid damage to the housing insert 8 by excessive temperatures.

Thus, to achieve a certain temperature T1 at the inlet mesh 85, the amount of aerosol generated can be calculated bearing in mind the losses experienced by the bulk aerosol travelling the distance d to the inlet mesh 85. This can be determined empirically, or through modelling of the system for a given target temperature T1 for the mesh 85. Hence, for a system in which the temperature T1 of the surface of the insert 8 is to be heated to between 50 °C to 150 °C, a balance on the amount of power P applied to the heater 48 to increase the temperature T1 of the inlet mesh 85 to improve the flavour and/or nicotine release versus the energy efficiency / lifetime of the device, sensory response, and undesired heating effects in conjunction with the distance d between the heater 48 and the surface of the insert 8 should be made for any given system.

Table 1 shows data obtained for two e-liquid formulations used for a specific implementation of the hybrid aerosol provision system 1. In this example, the aerosol provision system is largely as described in relation to Figures 1 and 2. The distance d was set at 6.2 mm, while the power supplied to the heater was set at 7.5 Watts for the results shown in Table 1 below.

The liquid formulations used in the test data below comprise propylene glycol (PG), vegetable glycerol (VG), water and a flavour component. The flavour component is largely negligible. The first formulation (Formulation 1 in the table) comprises around 71wt% PG, 17wt% VG and 12wt% water, while the second formulation (Formulation 2 in the table) comprises around 54wt% PG, 36wt% VG and 10wt% water. Tobacco was provided in the tobacco insert 8, having a total mass of 380 mg.

The test method involved simulating a block of 50 puffs using a 55 ml puff volume and a 3 second puff duration, with each puff separated by intervals of 30 seconds. An initial 1 second pre-puff activation of the heater 48 was also applied for each puff. This puffing regime is consistent with the Coresta Recommend Method Number 81 (CRM 81). This test was conducted on a Brogwaldt four-port smoke machine.

The measurements made below were obtained by identifying the maximum temperature over the 50 puffs.

**Table 1**

| Liquid Cartomiser and Cartomiser No. | Outlet Mesh Max Temperature (°C) | Inlet Mesh Max Temperature (°C) |
|---|---|---|
| Formulation 1 - Trial 1 | 54 | 93 |
| Formulation 1 - Trial 2 | 57 | 86 |
| Formulation 1 - Trial 3 | 57 | 110 |
| Formulation 2 - Trial 1 | 53 | 97 |
| Formulation 2 - Trial 2 | 56 | 119 |
| Formulation 2 - Trial 3 | 55 | 123 |

As can be seen from the table, the maximum temperatures of the inlet mesh 85 for all of the liquid tested fell in the range of 85 °C to 125 °C. As mentioned, the e-liquid composition can itself play a part in the maximum temperature of the inlet mesh 85. The first liquid generally yielded a lower average temperature (of around 96 °C) whereas the second liquid yielded a higher temperature (of around 113 °C).

Thus, it has been described above that the temperature of the lower surface of an aerosol modifying material part (insert 8) comprising an aerosol modifying material, such as tobacco, which is the surface closest to the heater 48 can be set so as to be between 50 °C to 150 °C, or between 70 °C to 140 °C, or between 85 °C to 125 °C, so as to provide a good sensory performance to a user using the device, while making efficient use of the available power for generating aerosol. The temperature of the lower surface can be set to within this range by appropriately setting the amount of aerosol to be generated per puff and/or by appropriately setting the distance between the heater and the lower surface of the aerosol modifying material part.

In addition to the above, it should also be noted that to minimise the thermal losses as the aerosol travels along air path 52, the air path 52 extends along a substantially straight line. In other words, the aerosol does not experience a sudden change of direction (e.g., by travelling around a corner), which may cause some of the aerosol to collide with the walls of the air path and deposit energy thus generally leading to a cooling of the aerosol.

In addition, it is considered that the thermal losses on average when travelling along an air path 52 will be reduced when increasing the amount of aerosol that is generated per puff. In this regard, it is thought that the greater the bulk material that is generated, the more on average the heat is retained within the bulk (bearing in mind that the air flow path 52 is of a fixed size).

In addition, the tobacco insert 8 is a component that is intended to be replaced / disposed of after a number of uses. Such components, when not refillable, are therefore required to be manufactured at a relatively high rate and to ideally to be made as cheaply as possible to provide a cost benefit to the user of the hybrid system 1. As mentioned above, the insert 8 may be formed largely from a plastics material, and in the described implementations, the plastics material is polypropylene. Polypropylene is considered suitable for use due to its abundance and easy manufacturability. The melting point of polypropylene is around 130 °C, and so setting the temperature T1 of the lower surface of the tobacco insert to less than 130 °C provides the added benefit that a cheaper tobacco insert 8 may be manufactured and used with the hybrid system 1. In alternative implementations, other plastic materials, potentially having a higher melting point, may also be used in accordance with the principles of the present disclosure.

While the above has focused on setting the temperature of the lower surface to be less than 150 °C, the temperature of the upper surface (e.g., the outlet mesh) may also desirably be set to within a range of temperatures. In particular, it is desirable for the aerosol exiting the insert 8 to not be too hot, as it is this aerosol that is inhaled by the user. For example, the temperature may be set to be less than 60 °C, for example.

Figure 3 is a schematic diagram showing a cross-section of a tobacco insert 8 to be used with the hybrid system 1 of Figure 1. The tobacco insert 8 is substantially the same as described previously, and similar components are indicated with similar reference numerals are not described in any further detail. The outlet 83 of the tobacco insert 8 includes an outlet mesh 86 which is separated by a small gap from the opening in the housing 81 of the tobacco insert 8. The outlet mesh 86 and the inlet mesh 85, along with the wall 81, effectively define an enclosed volume in which the tobacco material is held. That is, the tobacco material is held between the meshes 85 and 86. The distance between the two meshes is shown as d2 on Figure 3. The meshes 85 and 86 are shown as being generally flat, although it should be appreciated that the meshes may take any suitable shape such as domed, for example.

The temperature T2 of the outlet mesh 86 is again influenced by the aerosol passing though the mesh 86, much like as described for the inlet mesh 85. However, the temperature T2 of the outlet mesh 86 is expected to be less than the temperature T1 of the inlet mesh 85 due to the loss of energy to the tobacco itself.

As mentioned, the temperature of the outlet mesh 86 is desirably set such that the aerosol that exits the tobacco insert 8 via outlet 83 is not too hot for the user. The temperature of the aerosol when it enters the user's mouth should be around 50 °C or less. The temperature T2 can be set higher than this in some implementations due to the natural loss of energy as the aerosol propagates through space. Accordingly, the temperature T2 can be set to any suitable value provided there is sufficient distance from the mesh 86 to the outlet opening to allow the aerosol to cool to a temperature around 50 °C or less.

In Figure 3, the distance from the outlet to the mesh 86 is relatively short and so the temperature T2 is set to around 50 °C to 60 °C in this implementation. See Table 1 for example, where the outlet maximum temperature is shown as falling within this range.

Accordingly, the temperature T2 can be altered by setting an appropriate distance d2 between the upper and lower meshes and/or by adjusting the temperature T1 of the lower mesh 85. The temperature T1 and the distance d2 can be determined empirically or via computer modelling.

The distance d2 can be set within a suitable range. Setting the distance d2 too high may lead to loss of aerosol exiting the insert 8 as the aerosol may cool sufficiently to condensate on the tobacco itself. Setting the distance d2 too low may lead to a higher temperature aerosol exiting the insert 8. However, it should be appreciated that the temperature T1 can be controlled in accordance with any of the principles described above to account for distances d2 that do not provide a suitable temperature at the outlet of the tobacco insert 8. However, this may be to the detriment of sensory performance of the hybrid device 1.

In the present example, it has been found that a distance d2 of 15 mm, a surface A of the lower mesh of 72.54 mm², and a tobacco having a density of between 0.68-0.72 g/cc is suitable for providing an upper mesh temperature T2 of between 30 °C to 50 °C and a lower mesh temperature of between 85 °C to 125 °C.

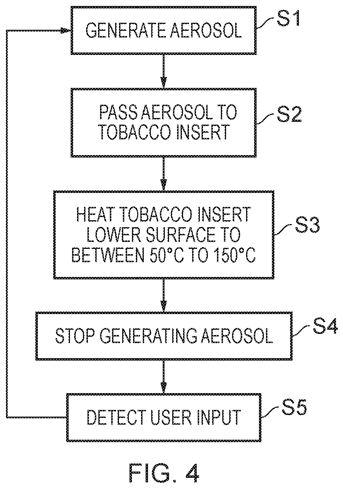

Figure 4 is an exemplary method for generating an aerosol according to the described implementations.

The method starts at step S1, where an aerosol is generated from the aerosol precursor material (e.g., e-liquid). As described above, this involves supplying power to the heater 48 to subsequently vaporise the e-liquid. The power may be set in advance taking into consideration the target temperature T1, distance to the lower surface of the aerosol modifying material part d, and the precursor composition.

The method proceeds to step S2 where the generated aerosol is passed to the aerosol modifying material part. This is typically performed by the user inhaling on the device causing air to flow along air flow path 52 taking the generated aerosol with it until it is delivered to the aerosol modifying material part. Here, as described, the aerosol performs two functions. On the one hand, the aerosol begins heating the lower surface of the aerosol modifying material part (see at step S3). On the other hand, the aerosol passes through the aerosol modifying material (e.g., tobacco) and is modified by the material, e.g., through entraining flavours and/or nicotine or other actives from the material. The aerosol is subsequently delivered to the user through the outlet 83.

At step S4, the aerosol generation is stopped. This may be as a result of sensing that the user has stopped inhaling (using pressure sensor 16) and/or that the user has stopped pressing button 14, or alternatively may be after a predetermined time has elapsed.

The method proceeds to step S5 where the control circuitry 20 detects a user input. As in step S4, the user input may be a signal received from pressure sensor 16 signifying a reduced pressure corresponding to a user inhaling on the device, and/or via detecting that the user has pushed button 14. Once this or a similar user input is detected, the control circuitry starts supplying power again to the heater and the method returns to step S1 and the process is repeated.

Thus, there has been described an aerosol provision system comprising an aerosol precursor material part comprising an aerosol precursor material to be vaporised; an aerosol modifying material part comprising an aerosol modifying material for modifying at least one property of a generated aerosol, wherein the aerosol modifying material part comprises a lower surface fluidly coupled to an aerosol pathway; and a heater for generating aerosol from the aerosol precursor material, the heater arranged in the aerosol pathway such that in normal use aerosol generated by the heater passes along the aerosol pathway to the aerosol modifying material part, wherein either the power supplied to the heater is set to generate a predetermined mass of aerosol per puff and the predetermined mass of aerosol generated per puff is set such that energy received at the lower surface of the aerosol modifying material part from the mass of aerosol causes the temperature of the lower surface of the aerosol modifying material part to be raised to between 50 °C to 150 °C, and/or the aerosol modifying material part is located at a distance from the heater such that the temperature of the lower surface of the aerosol modifying material part is set to be between 50 °C to 150 °C during normal use.

While the above-described embodiments have in some respects focussed on some specific example aerosol provision systems, it will be appreciated the same principles can be applied for aerosol provision systems using other technologies. That is to say, the specific manner in which various aspects of the aerosol provision system function are not directly relevant to the principles underlying the examples described herein.

For example, whereas the above-described embodiments have primarily focused on devices having an electrical heater based vaporiser for heating a liquid vapour precursor material, the same principles may be adopted in accordance with vaporisers based on other technologies, for example optical heating vaporisers, and also devices based on other aerosol precursor materials, for example solid materials, such as plant derived materials, such as tobacco derivative materials, or other forms of vapour precursor materials, such as gel, paste or foam based vapour precursor materials.

While it has generally been described that the tobacco pod 8 is insertable in / removable from the cartridge part 4, it should be appreciated that in other embodiments, the tobacco pod is integrally formed with the cartridge part 4. For example, the housing of the tobacco pod 8 may be integrally formed with the housing of the cartridge part 4. In such implementations, the tobacco pod 8 may be user-refillable (e.g., via provision of a closable hole that allows tobacco to be removed from and inserted into the inner volume of the tobacco pod), or may be non-refillable and thus cannot be changed / replaced independently of the cartridge part 4.

For example, the same principles may be adopted in an electronic cigarette which does not comprise a two-part modular construction, but which instead comprises a single-part device, for example a disposable (i.e. non-rechargeable and non-refillable) device. Furthermore, in some implementations of a modular device, the arrangement of components may be different. For example, in some implementations the control unit may also comprise the vaporiser with a replaceable cartridge providing a source of vapour precursor material for the vaporiser to use to generate vapour.

In order to address various issues and advance the art, this disclosure shows by way of illustration various embodiments in which the claimed invention(s) may be practiced. The advantages and features of the disclosure are of a representative sample of embodiments only, and are not exhaustive and / or exclusive. They are presented only to assist in understanding and to teach the claimed invention(s). It is to be understood that advantages, embodiments, examples, functions, features, structures, and / or other aspects of the disclosure are not to be considered limitations on the disclosure as defined by the claims

Various embodiments may suitably comprise, consist of, or consist essentially of, various combinations of the disclosed elements, components, features, parts, steps, means, etc. other than those specifically described herein.

## Claims

1. An aerosol provision system (1) comprising:
an aerosol precursor material part (4) comprising an aerosol precursor material to be vaporised;
an aerosol modifying material part (8) comprising an aerosol modifying material for modifying at least one property of a generated aerosol, wherein the aerosol modifying material part (8) comprises a lower surface (82, 85) fluidly coupled to an aerosol pathway (52); and
a heater (48) for generating aerosol from the aerosol precursor material, the heater (48) arranged in the aerosol pathway (52) such that in normal use aerosol generated by the heater (48) passes along the aerosol pathway (52) to the aerosol modifying material part (8),
wherein power supplied to the heater (48) is set to generate a predetermined mass of aerosol per puff, and
wherein the predetermined mass of aerosol generated per puff is set such that, accounting for energy losses from the aerosol while travelling from the heater (48) to the lower surface (82, 85) of the aerosol modifying material part (8), energy received at the lower surface (82, 85) of the aerosol modifying material part (8) from the mass of aerosol causes the temperature of the lower surface (82, 85) of the aerosol modifying material part (8) to be raised to between 50°C to 150°C.

2. The aerosol provision system (1) of claim 1, wherein the predetermined mass is set such that the temperature of the lower surface (82, 85) of the aerosol modifying material part (8) is raised to between 70°C to 140°C.

3. The aerosol provision system (1) of claim 1 or 2, wherein the predetermined mass is set such that the temperature of the lower surface (82, 85) of the aerosol modifying material part (8) is raised to between 85°C to 125°C.

4. The aerosol provision system (1) of any of the preceding claims, wherein the heater (48) is controlled so as to generate a pre-determined mass of between 4 to 12 milligrams per puff of aerosol from an aerosol generating material.

5. The aerosol provision system (1) of any of the preceding claims, wherein the predetermined mass of aerosol to be generated per puff is set in consideration of the distance between the heater (48) and the lower surface (82, 85) of the aerosol modifying material part (8) and/or wherein the temperature of the heater (48) is between 180°C to 260°C during normal use.

6. An aerosol provision system (1) comprising:
an aerosol precursor material part (4) comprising an aerosol precursor material to be vaporised;
an aerosol modifying material part (8) comprising an aerosol modifying material for modifying at least one property of a generated aerosol, wherein the aerosol modifying material part (8) comprises a lower surface (82, 85) fluidly coupled to an aerosol pathway (52); and
a heater (48) for generating aerosol from the aerosol precursor material, the heater (48) arranged in the aerosol pathway (52) such that in normal use aerosol generated by the heater (48) passes along the aerosol pathway (52) to the aerosol modifying material part (8),
wherein the heater (48) is configured to operate at a temperature of between 180°C to 260°C, and
wherein the aerosol modifying material part (8) is located at a distance from the heater (48) such that the temperature of the lower surface (82, 85) of the aerosol modifying material part (8), heated by the generated aerosol and / or the heater (48) for generating aerosol, is set to be between 50°C to 150°C during normal use.

7. The aerosol provision system (1) of claim 6, wherein the aerosol modifying material part (8) is located at a distance from the heater (48) such that the temperature of the lower surface (82, 85) of the aerosol modifying material part (8) is raised to between 70°C to 140°C or to between 85°C to 125°C.

8. The aerosol provision system (1) of any of claims 6 to 7, wherein the distance between the lower surface (82, 85) of the aerosol modifying material part (8) and the heater (48) is determined based on a predetermined mass of aerosol to be generated per puff.

9. The aerosol provision system (1) of any of the preceding claims, wherein the distance between the heater (48) and the lower surface of the aerosol modifying material part (8) is between 3 mm to 10 mm, and / or wherein the aerosol pathway (52) between the heater (48) and the lower surface (82, 85) of the aerosol modifying material part (8) extends along a straight line.

10. The aerosol provision system (1) of any of the preceding claims, wherein the power supplied to the heater (48) is between 6 W and 9 W, preferably between 6.5 W and 8.5 W, and / or wherein the heater (48) is made from an electrically conductive material such as a Nickel Chromium alloy, and / or the aerosol provision system (1) further comprises a wicking material (46), wherein the aerosol precursor material comprises a liquid, and the wicking material (46) is arranged to transport the liquid to the heater (48), wherein optionally, the liquid aerosol precursor material comprises one or more of the following: propylene glycol, vegetable glycerol, water, flavour, and active ingredients.

11. The aerosol provision system (1) of any of the preceding claims, wherein the aerosol modifying material comprises or consists of tobacco, wherein optionally the tobacco is tobacco granules.

12. The aerosol provision system (1) of any of the preceding claims, wherein the aerosol modifying material part (8) comprises a housing (81) for storing the aerosol modifying material, and wherein the housing (81) is formed from a polypropylene material, and / or wherein the aerosol modifying material part (8) comprises a housing (81) for storing the aerosol modifying material, wherein the housing comprises a first mesh (85) as the lower surface (82, 85) of the aerosol modifying material part (8), wherein optionally, the aerosol modifying material part (8) further comprises an upper surface (83) opposite the lower surface, and wherein further optionally the distance between the upper and lower surface (82, 83) is between 10 to 20 mm.

13. An aerosol provision device (2) for use with an aerosol provision system (1) further comprising an aerosol precursor material part (4) comprising an aerosol precursor material to be vaporised and an aerosol modifying material part (8) comprising an aerosol modifying material for modifying at least one property of a generated aerosol, wherein the aerosol modifying material part (8) comprises a lower surface (82, 85), the device (2) comprising:
control circuitry (20) for supplying power to a heater (48), the heater (48) for generating aerosol from the aerosol precursor material, the aerosol for passing through an aerosol pathway (52) to the lower surface (82, 85) of the aerosol modifying material part (8),
wherein power supplied to the heater (48) is set to generate a predetermined mass of aerosol per puff, and
wherein the predetermined mass of aerosol generated per puff is set such that, accounting for energy losses from the aerosol while travelling from the heater (48) to the lower surface (82, 85) of the aerosol modifying material part (8), energy received at the lower surface (82, 85) of the aerosol modifying material part (8) from the mass of aerosol causes the temperature of the lower surface (82, 85) of the aerosol modifying material part (8) to be raised to between 50°C to 150°C.

14. A cartridge part (4) for use with an aerosol provision system (1) further comprising an aerosol provision device (2), the cartridge part (4) comprising:
an aerosol precursor material part (4) comprising an aerosol precursor material to be vaporised;
an aerosol modifying material part (8) comprising an aerosol modifying material for modifying at least one property of a generated aerosol, wherein the aerosol modifying material part (8) comprises a lower surface (82, 85) fluidly coupled to an aerosol pathway (52); and
a heater (48) for generating aerosol from the aerosol precursor material, the heater (48) arranged in the aerosol pathway (52) such that in normal use aerosol generated by the heater (48) passes along the aerosol pathway (52) to the aerosol modifying material part (8),
wherein, in normal use, aerosol generated by the heater (48) passes along the aerosol pathway (52) to the aerosol modifying material part (8),
wherein the aerosol modifying material part (8), when coupled to the aerosol provision device (2), is configured so as to be located at a distance from the heater (48) such that the temperature of the lower surface (82, 85) of the aerosol modifying material part (8), heated by the generated aerosol and / or heater (48) for generating aerosol, is set to be between 50°C to 150°C during normal use.

15. A method of generating an aerosol comprising:
generating an aerosol from an aerosol precursor material to be vaporised by heating the aerosol precursor material using a heater (48);
passing the generated aerosol to an aerosol modifying material part (8) comprising an aerosol modifying material for modifying at least one property of the generated aerosol, wherein the aerosol modifying material part (8) comprises a lower surface (82, 85) fluidly coupled to an aerosol pathway (52),
heating, using the generated aerosol, the lower surface (82, 85) of the aerosol modifying material part (8) to a temperature between 50°C to 150°C.

## Patentansprüche

1. Aerosolabgabesystem (1), umfassend:
einen Aerosolvorläufermaterialteil (4), der ein zu verdampfendes Aerosolvorläufermaterial umfasst;
einen Aerosolmodifikationsmaterialteil (8), der ein Aerosolmodifikationsmaterial zum Modifizieren mindestens einer Eigenschaft eines erzeugten Aerosols umfasst, wobei der Aerosolmodifikationsmaterialteil (8) eine Unterseite (82, 85) umfasst, die strömungstechnisch mit einem Aerosolweg (52) verbunden ist; und
eine Heizvorrichtung (48) zum Erzeugen von Aerosol aus dem Aerosolvorläufermaterial, wobei die Heizvorrichtung (48) im Aerosolweg (52) derart angeordnet ist, dass im Normalbetrieb von der Heizvorrichtung (48) erzeugtes Aerosol entlang des Aerosolwegs (52) zum Aerosolmodifikationsmaterialteil (8) strömt,
wobei die der Heizvorrichtung (48) zugeführte Leistung so eingestellt ist, dass pro Ausstoß eine vorbestimmte Aerosolmasse erzeugt wird, und
wobei die pro Ausstoß erzeugte vorbestimmte Aerosolmasse so eingestellt ist, dass unter Berücksichtigung von Energieverlusten des Aerosols auf seinem Weg von der Heizvorrichtung (48) zur Unterseite (82, 85) des Aerosolmodifikationsmaterialteils (8) an der Unterseite (82, 85) des Aerosolmodifikationsmaterialteils (8) von der Aerosolmasse aufgenommene Energie bewirkt, dass die Temperatur der Unterseite (82, 85) des Aerosolmodifikationsmaterialteils (8) auf zwischen 50 °C und 150 °C erhöht wird.

2. Aerosolabgabesystem (1) nach Anspruch 1, wobei die vorbestimmte Masse so eingestellt ist, dass die Temperatur der Unterseite (82, 85) des Aerosolmodifikationsmaterialteils (8) auf zwischen 70 °C und 140 °C erhöht wird.

3. Aerosolabgabesystem (1) nach Anspruch 1 oder 2, wobei die vorbestimmte Masse so eingestellt ist, dass die Temperatur der Unterseite (82, 85) des Aerosolmodifikationsmaterialteils (8) auf zwischen 85 °C und 125 °C erhöht wird.

4. Aerosolabgabesystem (1) nach einem der vorhergehenden Ansprüche, wobei die Heizvorrichtung (48) so gesteuert wird, dass eine vorbestimmte Masse von 4 bis 12 Milligramm pro Aerosolausstoß aus einem aerosolerzeugenden Material erzeugt wird.

5. Aerosolabgabesystem (1) nach einem der vorhergehenden Ansprüche, wobei die pro Ausstoß zu erzeugende vorbestimmte Aerosolmasse unter Berücksichtigung des Abstands zwischen der Heizvorrichtung (48) und der Unterseite (82, 85) des Aerosolmodifikationsmaterialteils (8) eingestellt ist und/oder wobei die Temperatur der Heizvorrichtung (48) während des Normalbetriebs zwischen 180 °C und 260 °C liegt.

6. Aerosolabgabesystem (1), umfassend:
einen Aerosolvorläufermaterialteil (4), der ein zu verdampfendes Aerosolvorläufermaterial umfasst;
einen Aerosolmodifikationsmaterialteil (8), der ein Aerosolmodifikationsmaterial zum Modifizieren mindestens einer Eigenschaft eines erzeugten Aerosols umfasst, wobei der Aerosolmodifikationsmaterialteil (8) eine Unterseite (82, 85) umfasst, die strömungstechnisch mit einem Aerosolweg (52) verbunden ist; und
eine Heizvorrichtung (48) zum Erzeugen von Aerosol aus dem Aerosolvorläufermaterial, wobei die Heizvorrichtung (48) im Aerosolweg (52) derart angeordnet ist, dass im Normalbetrieb von der Heizvorrichtung (48) erzeugtes Aerosol entlang des Aerosolwegs (52) zum Aerosolmodifikationsmaterialteil (8) strömt,
wobei die Heizvorrichtung (48) so konfiguriert ist, dass sie bei einer Temperatur zwischen 180 °C und 260 °C arbeitet, und
wobei sich der Aerosolmodifikationsmaterialteil (8) in einem solchen Abstand von der Heizvorrichtung (48) befindet, dass die Temperatur der Unterseite (82, 85) des Aerosolmodifikationsmaterialteils (8), die durch das erzeugte Aerosol und/oder die Heizvorrichtung (48) zur Erzeugung des Aerosols erwärmt wird, im Normalbetrieb zwischen 50 °C und 150 °C eingestellt ist.

7. Aerosolabgabesystem (1) nach Anspruch 6, wobei sich der Aerosolmodifikationsmaterialteil (8) in einem solchen Abstand von der Heizvorrichtung (48) befindet, dass die Temperatur der Unterseite (82, 85) des Aerosolmodifikationsmaterialteils (8) auf zwischen 70 °C und 140 °C oder auf zwischen 85 °C und 125 °C erhöht wird.

8. Aerosolabgabesystem (1) nach einem der Ansprüche 6 bis 7, wobei der Abstand zwischen der Unterseite (82, 85) des Aerosolmodifikationsmaterialteils (8) und der Heizvorrichtung (48) auf Grundlage einer vorbestimmten Aerosolmasse bestimmt wird, die pro Ausstoß erzeugt werden soll.

9. Aerosolabgabesystem (1) nach einem der vorhergehenden Ansprüche, wobei der Abstand zwischen der Heizvorrichtung (48) und der Unterseite des Aerosolmodifikationsmaterialteils (8) zwischen 3 mm und 10 mm beträgt und/oder wobei der Aerosolweg (52) zwischen der Heizvorrichtung (48) und der Unterseite (82, 85) des Aerosolmodifikationsmaterialteils (8) entlang einer geraden Linie verläuft.

10. Aerosolabgabesystem (1) nach einem der vorhergehenden Ansprüche, wobei die der Heizvorrichtung (48) zugeführte Leistung zwischen 6 W und 9 W, bevorzugt zwischen 6,5 W und 8,5 W, beträgt und/oder wobei die Heizvorrichtung (48) aus einem elektrisch leitfähigen Material wie beispielsweise einer Nickel-Chrom-Legierung hergestellt ist und/oder das Aerosolabgabesystem (1) ferner ein Dochtmaterial (46) umfasst, wobei das Aerosolvorläufermaterial eine Flüssigkeit umfasst und das Dochtmaterial (46) so angeordnet ist, dass es die Flüssigkeit zur Heizvorrichtung (48) transportiert, wobei wahlweise das flüssige Aerosolvorläufermaterial eines oder mehrere der Folgenden umfasst: Propylenglykol, pflanzliches Glycerin, Wasser, Aromastoffe und Wirkstoffe.

11. Aerosolabgabesystem (1) nach einem der vorhergehenden Ansprüche, wobei das Aerosolmodifikationsmaterial Tabak umfasst oder aus diesem besteht, wobei es sich bei dem Tabak wahlweise um Tabakgranulat handelt.

12. Aerosolabgabesystem (1) nach einem der vorhergehenden Ansprüche, wobei der Aerosolmodifikationsmaterialteil (8) ein Gehäuse (81) zum Aufnehmen des Aerosolmodifikationsmaterials umfasst und wobei das Gehäuse (81) aus einem Polypropylenmaterial gebildet ist und/oder wobei der Aerosolmodifikationsmaterialteil (8) ein Gehäuse (81) zum Aufnehmen des Aerosolmodifikationsmaterials umfasst, wobei das Gehäuse ein erstes Netz (85) als Unterseite (82, 85) des Aerosolmodifikationsmaterialteils (8) umfasst, wobei wahlweise der Aerosolmodifikationsmaterialteil (8) ferner eine der Unterseite gegenüberliegende Oberseite (83) umfasst und wobei ferner wahlweise der Abstand zwischen der Ober- und der Unterseite (82, 83) zwischen 10 und 20 mm beträgt.

13. Aerosolabgabevorrichtung (2) zur Verwendung mit einem Aerosolabgabesystem (1), das ferner einen Aerosolvorläufermaterialteil (4), der ein zu verdampfendes Aerosolvorläufermaterial umfasst, sowie einen Aerosolmodifikationsmaterialteil (8) umfasst, der ein Aerosolmodifikationsmaterial zum Modifizieren mindestens einer Eigenschaft eines erzeugten Aerosols umfasst, wobei der Aerosolmodifikationsmaterialteil (8) eine Unterseite (82, 85) umfasst, wobei die Vorrichtung (2) umfasst:
eine Steuerschaltungsanordnung (20) zum Zuführen von Leistung zu einer Heizvorrichtung (48), wobei die Heizvorrichtung (48) dazu dient, aus dem Aerosolvorläufermaterial ein Aerosol zu erzeugen, wobei das Aerosol durch einen Aerosolweg (52) zur Unterseite (82, 85) des Aerosolmodifikationsmaterialteils (8) geleitet wird,
wobei die der Heizvorrichtung (48) zugeführte Leistung so eingestellt ist, dass pro Ausstoß eine vorbestimmte Aerosolmasse erzeugt wird, und
wobei die pro Ausstoß erzeugte vorbestimmte Aerosolmasse so eingestellt ist, dass unter Berücksichtigung von Energieverlusten des Aerosols auf seinem Weg von der Heizvorrichtung (48) zur Unterseite (82, 85) des Aerosolmodifikationsmaterialteils (8) an der Unterseite (82, 85) des Aerosolmodifikationsmaterialteils (8) von der Aerosolmasse aufgenommene Energie bewirkt, dass die Temperatur der Unterseite (82, 85) des Aerosolmodifikationsmaterialteils (8) auf zwischen 50 °C und 150 °C erhöht wird.

14. Kartuschenteil (4) zur Verwendung mit einem Aerosolabgabesystem (1), das ferner eine Aerosolabgabevorrichtung (2) umfasst, wobei der Kartuschenteil (4) umfasst:
einen Aerosolvorläufermaterialteil (4), der ein zu verdampfendes Aerosolvorläufermaterial umfasst;
einen Aerosolmodifikationsmaterialteil (8), der ein Aerosolmodifikationsmaterial zum Modifizieren mindestens einer Eigenschaft eines erzeugten Aerosols umfasst, wobei der Aerosolmodifikationsmaterialteil (8) eine Unterseite (82, 85) umfasst, die strömungstechnisch mit einem Aerosolweg (52) verbunden ist; und
eine Heizvorrichtung (48) zum Erzeugen von Aerosol aus dem Aerosolvorläufermaterial, wobei die Heizvorrichtung (48) im Aerosolweg (52) derart angeordnet ist, dass im Normalbetrieb von der Heizvorrichtung (48) erzeugtes Aerosol entlang des Aerosolwegs (52) zum Aerosolmodifikationsmaterialteil (8) strömt,
wobei im Normalbetrieb von der Heizvorrichtung (48) erzeugtes Aerosol entlang des Aerosolwegs (52) zum Aerosolmodifikationsmaterialteil (8) strömt,
wobei der Aerosolmodifikationsmaterialteil (8), wenn er mit der Aerosolabgabevorrichtung (2) gekoppelt ist, so konfiguriert ist, dass er sich in einem solchen Abstand vom Heizelement (48) befindet, dass die Temperatur der Unterseite (82, 85) des Aerosolmodifikationsmaterialteils (8), die durch das erzeugte Aerosol und/oder die Heizvorrichtung (48) zur Erzeugung von Aerosol erwärmt wird, im Normalbetrieb zwischen 50 °C und 150 °C eingestellt ist.

15. Verfahren zum Erzeugen eines Aerosols, umfassend:
Erzeugen eines Aerosols aus einem Aerosolvorläufermaterial, das durch Erhitzen des Aerosolvorläufermaterials mittels einer Heizvorrichtung (48) verdampft werden soll;
Leiten des erzeugten Aerosols zu einem Aerosolmodifikationsmaterialteil (8), der ein Aerosolmodifikationsmaterial zum Modifizieren mindestens einer Eigenschaft des erzeugten Aerosols umfasst, wobei der Aerosolmodifikationsmaterialteil (8) eine Unterseite (82, 85) umfasst, die strömungstechnisch mit einem Aerosolweg (52) verbunden ist,
Erwärmen der Unterseite (82, 85) des Aerosolmodifikationsmaterialteils (8) unter Verwendung des erzeugten Aerosols auf eine Temperatur zwischen 50 °C und 150 °C.

## Revendications

1. Système de fourniture d'aérosol (1) comprenant :
une partie de matériau précurseur d'aérosol (4) comprenant un matériau précurseur d'aérosol devant être vaporisé ;
une partie de matériau modificateur d'aérosol (8) comprenant un matériau modificateur d'aérosol destiné à modifier au moins une propriété d'un aérosol généré, la partie de matériau modificateur d'aérosol (8) comprenant une surface inférieure (82, 85) couplée de manière fluidique à un passage d'aérosol (52) ; et
un élément chauffant (48) destiné à générer un aérosol à partir du matériau précurseur d'aérosol, l'élément chauffant (48) étant agencé dans le passage d'aérosol (52) de sorte que, en utilisation normale, l'aérosol généré par l'élément chauffant (48) passe le long du passage d'aérosol (52) jusqu'à la partie de matériau modificateur d'aérosol (8),
dans lequel la puissance fournie à l'élément chauffant (48) est fixée de manière à générer une masse prédéterminée d'aérosol par bouffée, et
dans lequel la masse prédéterminée d'aérosol générée par bouffée est fixée de sorte que, compte tenu des pertes d'énergie de l'aérosol au cours de son parcours depuis l'élément chauffant (48) jusqu'à la surface inférieure (82, 85) de la partie de matériau modificateur d'aérosol (8), l'énergie reçue au niveau de la surface inférieure (82, 85) de la partie de matériau modificateur d'aérosol (8) en provenance de la masse d'aérosol provoque une élévation de la température de la surface inférieure (82, 85) de la partie de matériau modificateur d'aérosol (8) jusqu'à une valeur comprise entre 50 °C et 150 °C.

2. Système de fourniture d'aérosol (1) selon la revendication 1, dans lequel la masse prédéterminée est fixée de sorte que la température de la surface inférieure (82, 85) de la partie de matériau modificateur d'aérosol (8) soit élevée jusqu'à une valeur comprise entre 70 °C et 140 °C.

3. Système de fourniture d'aérosol (1) selon la revendication 1 ou 2, dans lequel la masse prédéterminée est fixée de sorte que la température de la surface inférieure (82, 85) de la partie de matériau modificateur d'aérosol (8) soit élevée jusqu'à une valeur comprise entre 85 °C et 125 °C.

4. Système de fourniture d'aérosol (1) selon l'une quelconque des revendications précédentes, dans lequel l'élément chauffant (48) est commandé de manière à générer une masse prédéterminée comprise entre 4 et 12 milligrammes par bouffée d'aérosol à partir d'un matériau générateur d'aérosol.

5. Système de fourniture d'aérosol (1) selon l'une quelconque des revendications précédentes, dans lequel la masse prédéterminée d'aérosol devant être générée par bouffée est fixée compte tenu de la distance entre l'élément chauffant (48) et la surface inférieure (82, 85) de la partie de matériau modificateur d'aérosol (8) et/ou dans lequel la température de l'élément chauffant (48) est comprise entre 180 °C et 260 °C pendant une utilisation normale.

6. Système de fourniture d'aérosol (1) comprenant :
une partie de matériau précurseur d'aérosol (4) comprenant un matériau précurseur d'aérosol devant être vaporisé ;
une partie de matériau modificateur d'aérosol (8) comprenant un matériau modificateur d'aérosol destiné à modifier au moins une propriété d'un aérosol généré, la partie de matériau modificateur d'aérosol (8) comprenant une surface inférieure (82, 85) couplée de manière fluidique à un passage d'aérosol (52) ; et
un élément chauffant (48) destiné à générer un aérosol à partir du matériau précurseur d'aérosol, l'élément chauffant (48) étant agencé dans le passage d'aérosol (52) de sorte que, en utilisation normale, l'aérosol généré par l'élément chauffant (48) passe le long du passage d'aérosol (52) jusqu'à la partie de matériau modificateur d'aérosol (8),
dans lequel l'élément chauffant (48) est configuré pour fonctionner à une température comprise entre 180 °C et 260 °C, et
dans lequel la partie de matériau modificateur d'aérosol (8) est située à une distance de l'élément chauffant (48) telle que la température de la surface inférieure (82, 85) de la partie de matériau modificateur d'aérosol (8), chauffée par l'aérosol généré et/ou l'élément chauffant (48) destiné à générer un aérosol, est fixée de manière à être comprise entre 50 °C et 150 °C pendant une utilisation normale.

7. Système de fourniture d'aérosol (1) selon la revendication 6, dans lequel la partie de matériau modificateur d'aérosol (8) est située à une distance de l'élément chauffant (48) telle que la température de la surface inférieure (82, 85) de la partie de matériau modificateur d'aérosol (8) est élevée jusqu'à une valeur comprise entre 70 °C et 140 °C ou entre 85 °C et 125 °C.

8. Système de fourniture d'aérosol (1) selon l'une quelconque des revendications 6 à 7, dans lequel la distance entre la surface inférieure (82, 85) de la partie de matériau modificateur d'aérosol (8) et l'élément chauffant (48) est déterminée sur la base d'une masse prédéterminée d'aérosol devant être générée par bouffée.

9. Système de fourniture d'aérosol (1) selon l'une quelconque des revendications précédentes, dans lequel la distance entre l'élément chauffant (48) et la surface inférieure de la partie de matériau modificateur d'aérosol (8) est comprise entre 3 mm et 10 mm, et/ou dans lequel le passage d'aérosol (52) entre l'élément chauffant (48) et la surface inférieure (82, 85) de la partie de matériau modificateur d'aérosol (8) s'étend le long d'une ligne droite.

10. Système de fourniture d'aérosol (1) selon l'une quelconque des revendications précédentes, dans lequel la puissance fournie à l'élément chauffant (48) est comprise entre 6 W et 9 W, de préférence entre 6,5 W et 8,5 W, et/ou dans lequel l'élément chauffant (48) est constitué d'un matériau électriquement conducteur tel qu'un alliage nickel-chrome, et/ou le système de fourniture d'aérosol (1) comprend en outre un matériau à effet mèche (46), dans lequel le matériau précurseur d'aérosol comprend un liquide, et le matériau à effet de mèche (46) est agencé de manière à transporter le liquide jusqu'à l'élément chauffant (48), dans lequel, éventuellement, le matériau précurseur d'aérosol liquide comprend un ou plusieurs des éléments suivants : propylène glycol, glycérol d'origine végétale, eau, arôme et ingrédients actifs.

11. Système de fourniture d'aérosol (1) selon l'une quelconque des revendications précédentes, dans lequel le matériau modificateur d'aérosol comprend ou consiste en du tabac, dans lequel, éventuellement, le tabac est sous forme de granulés de tabac.

12. Système de fourniture d'aérosol (1) selon l'une quelconque des revendications précédentes, dans lequel la partie de matériau modificateur d'aérosol (8) comprend un boîtier (81) destiné à stocker le matériau modificateur d'aérosol, et dans lequel le boîtier (81) est formé d'un matériau en polypropylène, et/ou dans lequel la partie de matériau modificateur d'aérosol (8) comprend un boîtier (81) destiné à stocker le matériau modificateur d'aérosol, dans lequel le boîtier comprend une première grille (85) jouant le rôle de la surface inférieure (82, 85) de la partie de matériau modificateur d'aérosol (8), dans lequel, éventuellement, la partie de matériau modificateur d'aérosol (8) comprend en outre une surface supérieure (83) opposée à la surface inférieure, et dans lequel, éventuellement en outre, la distance entre les surfaces supérieure et inférieure (82, 83) est comprise entre 10 et 20 mm.

13. Dispositif de fourniture d'aérosol (2) destiné à être utilisé avec un système de fourniture d'aérosol (1) comprenant en outre une partie de matériau précurseur d'aérosol (4) comprenant un matériau précurseur d'aérosol destiné à être vaporisé et une partie de matériau modificateur d'aérosol (8) comprenant un matériau modificateur d'aérosol destiné à modifier au moins une propriété d'un aérosol généré, dans lequel la partie de matériau modificateur d'aérosol (8) comprend une surface inférieure (82, 85), le dispositif (2) comprenant :
une circuiterie de commande (20) destinée à fournir de la puissance à un élément chauffant (48), l'élément chauffant (48) étant destiné à générer un aérosol à partir du matériau précurseur d'aérosol, l'aérosol étant destiné à passer à travers un passage d'aérosol (52) jusqu'à la surface inférieure (82, 85) de la partie de matériau modificateur d'aérosol (8),
dans lequel la puissance fournie à l'élément chauffant (48) est fixée de manière à générer une masse prédéterminée d'aérosol par bouffée, et
dans lequel la masse prédéterminée d'aérosol générée par bouffée est fixée de sorte que, compte tenu des pertes d'énergie de l'aérosol au cours de son parcours depuis l'élément chauffant (48) jusqu'à la surface inférieure (82, 85) de la partie de matériau modificateur d'aérosol (8), l'énergie reçue au niveau de la surface inférieure (82, 85) de la partie de matériau modificateur d'aérosol (8) en provenance de la masse d'aérosol provoque une élévation de la température de la surface inférieure (82, 85) de la partie de matériau modificateur d'aérosol (8) jusqu'à une valeur comprise entre 50 °C et 150 °C.

14. Partie cartouche (4) destinée à être utilisée avec un système de fourniture d'aérosol (1) comprenant en outre un dispositif de fourniture d'aérosol (2), la partie cartouche (4) comprenant :
une partie de matériau précurseur d'aérosol (4) comprenant un matériau précurseur d'aérosol devant être vaporisé ;
une partie de matériau modificateur d'aérosol (8) comprenant un matériau modificateur d'aérosol destiné à modifier au moins une propriété d'un aérosol généré, la partie de matériau modificateur d'aérosol (8) comprenant une surface inférieure (82, 85) couplée de manière fluidique à un passage d'aérosol (52) ; et
un élément chauffant (48) destiné à générer un aérosol à partir du matériau précurseur d'aérosol, l'élément chauffant (48) étant agencé dans le passage d'aérosol (52) de sorte que, en utilisation normale, l'aérosol généré par l'élément chauffant (48) passe le long du passage d'aérosol (52) jusqu'à la partie de matériau modificateur d'aérosol (8),
dans laquelle, en utilisation normale, l'aérosol généré par l'élément chauffant (48) passe le long du passage d'aérosol (52) jusqu'à la partie de matériau modificateur d'aérosol (8),
dans laquelle la partie de matériau modificateur d'aérosol (8), lorsqu'elle est couplée au dispositif de fourniture d'aérosol (2), est configurée de manière à être située à une distance de l'élément chauffant (48) telle que la température de la surface inférieure (82, 85) de la partie de matériau modificateur d'aérosol (8), chauffée par l'aérosol généré et/ou l'élément chauffant (48) destiné à générer un aérosol, est fixée de manière à être comprise entre 50 °C et 150 °C pendant une utilisation normale.

15. Procédé de génération d'un aérosol comprenant :
la génération d'un aérosol à partir d'un matériau précurseur d'aérosol devant être vaporisé par chauffage du matériau précurseur d'aérosol au moyen d'un élément chauffant (48) ;
le passage de l'aérosol jusqu'à une partie de matériau modificateur d'aérosol (8) comprenant un matériau modificateur d'aérosol destiné à modifier au moins une propriété de l'aérosol généré, la partie de matériau modificateur d'aérosol (8) comprenant une surface inférieure (82, 85) couplée de manière fluidique à un passage d'aérosol (52),
le chauffage, au moyen de l'aérosol généré, de la surface inférieure (82, 85) de la partie de matériau modificateur d'aérosol (8) jusqu'à une température comprise entre 50 °C et 150 °C.
